# EUROPEAN PATENT SPECIFICATION

(11) **EP 2 301 444 B1**
(45) Date of publication and mention of the grant of the patent: **06.06.2012**
(21) Application number: 10184648.3
(22) Date of filing: 20.10.2001
(51) Int. Cl.: A61B 17/00, A61B 17/34, A61B 17/02

(54) **Wound retraction apparatus**
Wundretraktionsvorrichtung
Appareil pour retraction de plaies

(43) Date of publication of application: 30.03.2011
(62) Divisional of application: 01985170.8
(73) Proprietor: APPLIED MEDICAL RESOURCES CORPORATION, Rancho Santa Margarita, CA 92688 (US)
(72) Inventor: Ewers, Richard C, Fullerton, CA 92833 (US); Brustad, John R, Dana Point, CA 92629 (US); Pingleton, Edward D, Laguna Niguel, CA 92677 (US); Halal, Nabil, Laguna Niguel, CA 92677 (US); Adlparvar, Payam, Lake Forest, CA 92630 (US); Taylor, Scott, Mission Viejo, CA 92692 (US); Dulak, Gary R, Newport Beach, CA 92663 (US); Dunn, Michael J, Santa Ana, CA 92706 (US); Morales, Norman, San Jose, CA 95126 (US); Hart, Charles C, Summerville, SC 29483-8949 (US); Bowes, Robert R ,II, Trabuco Canyon, CA 92679 (US)
(74) Representative: Fitchett, Stuart Paul

(56) References cited:
- EP-A- 1 125 552
- EP-A1- 1 407 715
- WO-A-00/32116
- US-A- 5 514 133

## Description

### Background of the Invention

### Field of Invention

This invention relates generally to wound retraction and more specifically to wound retraction in a laparoscopic surgical procedure.

### Cross Reference to Related Applications

This is a divisional application of European Patent Application No. 01985170.8.

### Discussion of the Related Art

During laparoscopic surgery, it is desirable to inflate the abdominal cavity in order to increase the volume of the working space. This is accomplished with an insufflation gas which must be maintained at a pressure sufficient to inflate the abdomen. Maintaining the pressure of the insufflation gas is difficult when it is also desirable to insert instrumentation through the abdominal wall. If the surgeon is interested in inserting his or her hand in a hand-assisted laparoscopic procedure, the maintenance of insufflation pressure is even more difficult. Currently, several devices exist that accomplish this surgical need although they suffer from drawbacks such as difficult placement and cumbersome use. Thus, it is desirable that the wound be retracted, protected, and fixed while maintaining an insufflation seal.

European Patent Application, Publication No. EP 1125552A1 discloses a retractor where the sheath may be anchored to the outer ring to adjust the length of the sheath.

EP 5 514 133 on which the two-part form is based equally discloses a surgical wound retractor.

### Summary of the Invention

The invention provides a surgical wound retractor according to claim 1 adapted to dilate a wound, the retractor comprising:
a sheath having a generally cylindrical configuration;
an inner ring fixed to the sheath, the inner ring having a diameter greater than a desired diameter of the wound and being adapted for disposition interiorly of the wound; and
an outer ring having a diameter greater than the desired diameter of the wound and adapted for disposition exteriorly of the wound, wherein the outer ring has a plurality of hook features on it and the inner ring has a plurality of elastic tensioners attached to it which in use can be pulled taut and secured to respective hook features so that, in use, the hook features allow the user to fix the retractor in position to different sizes of abdominal walls.

A wound retraction in accordance with the present invention allows the surgeon to easily locate a retractor and to provide a solid base for an instrument or hand seal. This retractor removes the tissue pressure from the wrist during hand-assisted laparoscopic surgery. It can also protect the tissue at the wound site, for example, from abrasion, bacteria or other contaminants organs, such as donor kidneys to be removed with minimal risk or damage. The retractor also opens the wound providing greater access to the operative site for instruments, such as the hand of the surgeon. The sheath may be an elastic material.

These and other features and advantages of the invention will become more apparent with a description of a preferred embodiment and reference to the associated drawings.

### Description of the Drawings

FIGS. and 2 are perspective axial cross sections, illustrating the concept of protecting and retracting a wound site with a wound retractor of the present invention.

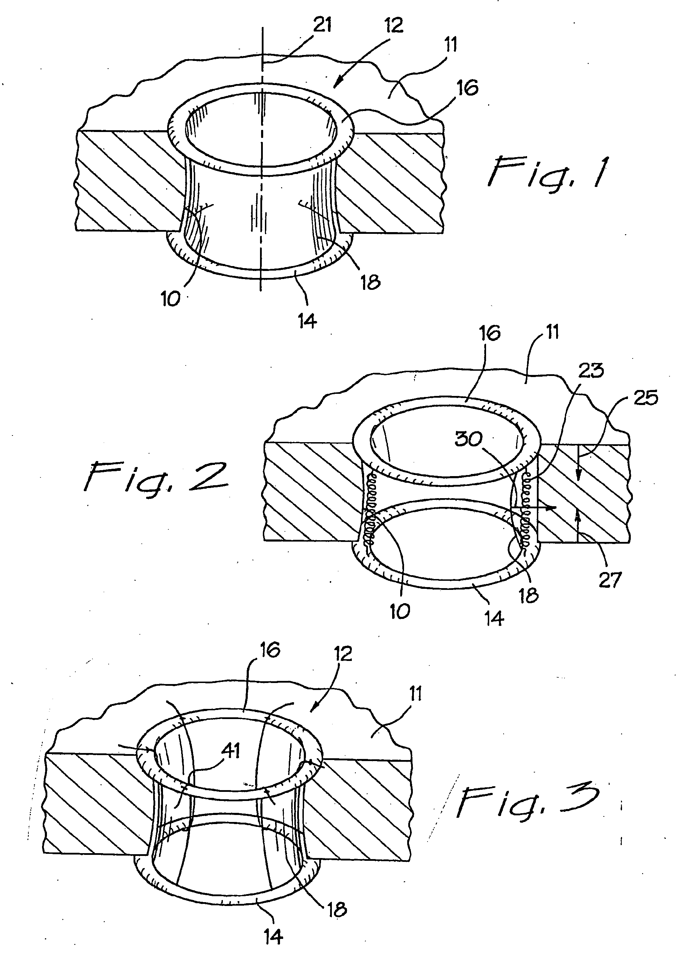

FIG. 3 is a perspective view of a retractor in accordance with the present invention.

### Description of Preferred Embodiments and Best Mode of the Invention

The basic concept of retracting and protecting a wound site is illustrated in the prospective view of Figure 1 wherein a wound 10 is formed in an abdominal wall 11. In this embodiment, a retractor 12 uses two rings 14 and 16 which are fixed to an elastic sheath 18. The sheath 18 has a generally cylindrical configuration and is disposed along an axis 21. The rings 14 and 16 are disposed in respective planes which extend radially of the axis 21.

The sheath 18 has elastomeric properties, but in its natural, unstretched state the two rings 14 and 16 are separate by a natural distance. The lower ring 14 is placed interiorly of the abdominal wall 11 and the upper ring 14 is stretched beyond the natural distance away from the lower ring. Once the elastic sheath 18 has been stretched to a distance greater than the abdominal wall thickness, the upper ring 16 is placed on the surface of the skin.

Since the diameters of the rings 14, 16 are greater than that desired for the wound site 10, they will have sufficient footing to maintain this tension between the two rings 14, 16. This tension is created by the elastic material that has been stretched and retained at a distance greater than the natural distance. It will be appreciated that in many embodiments, the sheath 18 can be formed of a non-elastic sheathing material. In a similar manner, the rings 14 and 16 may be provided with a rigid configuration or alternatively may be formed of an elastromeric material.

Figure 2 shows a simple schematic of the ring dynamics illustrated in Figure 1, with the retractor 12 operatively disposed across the abdominal wall 11. The elastic sheet sheath 18 acts as a circumferential spring 23 around the wound site 10 that evenly distributes the tension between the two rings 14 and 16, as represented by arrows 25 & 27. In addition, the elastic sheeting provides a radial retraction force 30 around the wound to enlarge the wound site 10 in order to facilitate the passage of instruments, such as the hand of the surgeon.

The amount of tension force between the two rings 14 and 16 can be controlled by the elastomeric proportion of the elastic sheath 18. In order to accommodate a larger range of abdominal wall thicknesses, a material with a higher elasticity can be chosen to allow for greater stretch.

The Figure 3 embodiment, in accordance with the present invention, provides even further versatility. This design is not limited by the fixed distance between the rings 14, 16 of the retractor 12. Instead, the inner ring 14 will be fixed to the sheath 18, and the upper ring 16 will comprise a separate assembly. Initially, the inner ring 14 is placed into the peritoneum cavity. Then the elastic material is pulled taut and secured onto hook features 41 located on the outer ring 16. This allows the user to fix the retractor 12 to an abdominal wall of any size.

Those skilled in the art may envision modifications within the scope of the present invention as defined by the following claims.

## Claims

1. A surgical wound retractor (12) adapted to dilate a wound, the retractor comprising:
a sheath (18) having a generally cylindrical configuration;
an inner ring (14) fixed to the sheath (18), the inner ring (14) having a diameter greater than a desired diameter of the wound and being adapted for disposition interiorly of the wound; and
an outer ring (16) having a diameter greater than the desired diameter of the wound and adapted for disposition exteriorly of the wound wherein the outer ring (16) has a plurality of hook features (41) on it and the inner ring (14) has a plurality of tensioners attached to it which in use can be pulled taut and secured to respective hook features (41) so that the hook features allow the user to fix the retractor in position to different sizes of abdominal walls **characterised in that** the tensioners are elastic tensioners.

2. The retractor of claim 1 wherein the distance separating the first ring from the second ring is adjustable.

3. The retractor of claims 1or 2 wherein the retraction force is based on the distance separating the first ring and the second ring.

4. The retractor of claim 1, 2 or 3 wherein the upper ring (16) comprises a separate assembly to the lower ring.

5. The retractor of any preceding claim wherein the sheath is an elastic material.

6. The retractor of any preceding claim wherein the elastic tensioners are located inside the sheath (18).

## Patentansprüche

1. Ein chirurgischer Wundretraktor (12), der zur Dilatation einer Wunde angepasst ist, wobei der Retraktor umfasst:
eine Schleuse (18), die eine allgemein zylinderförmige Konfiguration aufweist;
einen Innenring (14), der an der Schleuse (18) befestigt ist, wobei der Innenring (14) einen Durchmesser aufweist, der größer als ein gewünschter Durchmesser der Wunde ist und zur Ablage innerhalb der Wunde angepasst ist; und
einen Außenring (16), der einen Durchmesser aufweist, der größer ist als der gewünschte Durchmesser der Wunde und zur Ablage außerhalb der Wunde angepasst ist, **gekennzeichnet dadurch, dass** die Spannvorrichtungen elastische Spannvorrichtungen sind, wobei sich auf dem Außenring (16) eine Vielzahl von Hakenelementen (41) befinden und an dem Innenring (14) eine Vielzahl von Spannvorrichtungen angebracht sind, die fest angezogen und an den jeweiligen Hakenelementen (41) gesichert werden können, so dass die Hakenelemente es dem Anwender erlauben, den Retraktor an verschiedenen Größen von Bauchwänden an Ort und Stelle zu fixieren.

2. Der Retraktor nach Anspruch 1, wobei der Abstand, der den ersten Ring von dem zweiten Ring trennt, anpassbar ist.

3. Der Retraktor nach Anspruch 1 oder 2, wobei die Retraktionskraft auf dem Abstand basiert, der den ersten Ring und den zweiten Ring voneinander trennt.

4. Der Retraktor nach Anspruch 1, 2 oder 3, wobei der obere Ring (16) eine gegenüber dem unteren Ring separate Baugruppe umfasst.

5. Der Retraktor nach einem beliebigen der vorhergehenden Ansprüche, wobei die Schleuse ein elastisches Material ist.

6. Der Retraktor nach einem beliebigen der vorhergehenden Ansprüche, wobei die elastischen Spannvorrichtungen sich im Innern der Schleuse (18) befinden.

## Revendications

1. Rétracteur de plaie chirurgicale (12) adapté pour dilater une plaie, le rétracteur comprenant :
une gaine (18) ayant une configuration généralement cylindrique ;
un anneau interne (14) fixé à la gaine (18), l'anneau interne (14) ayant un diamètre supérieur à un diamètre désiré de la plaie et étant adapté de manière à être disposé intérieurement à la plaie ; et
un anneau externe (16) ayant un diamètre supérieur au diamètre désiré de la plaie et adapté de manière à être disposé extérieurement à la plaie, dans lequel l'anneau extérieur (16) porte une pluralité d'éléments en forme de crochet (41) et l'anneau interne (14) porte une pluralité de tensionneurs qui y sont fixés et qui, durant l'utilisation, peuvent être tirés jusqu'à raidissement et fixés de manière sûre aux éléments en forme de crochet (41) respectifs, de telle sorte que les éléments en forme de crochet permettent à l'utilisateur de fixer le rétracteur en position à différentes tailles de parois abdominales, **caractérisé en ce que** les tensionneurs sont des tensionneurs élastiques.

2. Rétracteur selon la revendication 1, dans lequel la distance séparant le premier anneau du second anneau est ajustable.

3. Rétracteur selon la revendication 1 ou 2, dans lequel la force de rétraction est fonction de la distance séparant le premier anneau et le second anneau.

4. Rétracteur selon la revendication 1, 2 ou 3, dans lequel l'anneau supérieur (16) comprend un ensemble séparé de l'anneau inférieur.

5. Rétracteur selon l'une quelconque des revendications précédentes, dans lequel la gaine est en un matériau élastique.

6. Rétracteur selon l'une quelconque des revendications précédentes, dans lequel les tensionneurs élastiques sont situés à l'intérieur de la gaine (18).
